# EUROPÄISCHE PATENTSCHRIFT

(11) **EP 1 984 033 B1**
(45) Veröffentlichungstag und Bekanntmachung des Hinweises auf die Patenterteilung: **29.06.2011**
(21) Anmeldenummer: 07711551.7
(22) Anmeldetag: 15.02.2007
(51) Int. Cl.: A61L 27/30, A61L 31/08

(54) **VERFAHREN ZUR UMHÜLLUNG EINES STENTS**
METHOD FOR WRAPPING A STENT
PROCÉDÉ D'ENVELOPPEMENT D'UN STENT

(30) Priorität: 15.02.2006 DE 102006007231
(43) Veröffentlichungstag der Anmeldung: 29.10.2008
(73) Patentinhaber: Acandis GmbH & Co. KG, 76327 Pfinztal (DE)
(72) Erfinder: KOCH, Michael, 53225 Bonn (DE); QUANDT, Eckhard, 24226 Heikendorf (DE); RUMPF, Holger, 72768 Reutlingen (DE); ZAMPONI, Christiane, 24114 Kiel (DE)
(74) Vertreter: Kilchert, Jochen
(86) Internationale Anmeldenummer: PCT/EP2007/001329
(87) Internationale Veröffentlichungsnummer: WO 2007/093423

(56) Entgegenhaltungen:
- EP-A- 1 479 358
- WO-A-01/87371
- DE-A1- 19 951 279
- US-A- 6 019 784
- US-A1- 2003 060 873

## Beschreibung

Die vorliegende Erfindung betrifft ein Verfahren zur Aufbringung einer bevorzugt metallischen Abdeckschicht auf einem röhrchenförmigen endoluminalen Implantat, insbesondere einer Gefäßstütze (Stent), wobei die Oberfläche des Implantats von einer Vielzahl von Ausnehmungen durchbrochen ist, wobei die Abdeckschicht durch Abscheidung von Material auf der Oberfläche des Implantats erzeugt wird. Die Erfindung betrifft ebenso das nach diesem Verfahren umhüllte Implantat.

Stents, die kleinen röhrchenförmigen Gittergerüsten gleichen, werden bei der therapeutischen Erweiterung von Gefäßen, beispielsweise der Herzkranzgefäße, oder zur Vorbeugung von erneut auftretenden Verengungen in die Gefäße implantiert und stabilisieren dort die Gefäßwände. Die gitterartige Struktur der Stents ist notwendig, damit diese in einem zusammengefalteten Zustand in das Gefäß eingeführt werden können, bevor sie entfaltet und damit von Innen an die Wand eines Gefäßes angelegt werden können. Zu unterscheiden sind einerseits selbst entfaltende Stents, die sich federartig entspannen und dabei entfalten, nachdem vor Ort eine umgebende Plastikhülle abgezogen wurde. Im Gegensatz dazu werden andere Stents durch ein Ballon-Katheder platziert und aufgeweitet.

Problematisch an den bislang bekannten Stents ist, dass ihre gitterartige Struktur zu einer Reizung des Gewebes und daher gewissermaßen zu einer Verletzung der Gefäßinnenwand führen kann. Bei der Heilung dieser endogenen Entzündung entsteht Gewebe, das die Ausnehmungen der Oberfläche durchdringt und in das Lumen hineinwächst. In etwa 20% der Fälle wird das Gefäß durch diesen Prozess der Restenose wieder so weit verengt, dass ein erneuter Eingriff notwendig ist.

Aus der DE 199 51 279 A1 ist ein Stent bekannt, der mehrere Ringe umfasst, die die Entfaltung bzw. eine Expansion des Gittergerüsts des Stents begrenzen.

Es sind auch sogenannte "covered" Stents bekannt, die mit einem Netz aus PTFE umgeben sind, wobei die Umhüllung zur Verminderung der Restenose beitragen soll. Ein weiterer vorteilhafter Effekt der Umhüllung ist, dass das in den Gefäßen befindliche thrombotische Material durch die weiter geschlossene Oberfläche des Stents an die Innenwandung des Gefäßes gedrückt und dort festgehalten wird. Bei der Implantation des Stents kann somit die Einbringung des thrombotischen Materials in den Blutfluss weitgehend vermieden werden, so dass die Gefahr der Verbreitung dieses Materials im Kreislauf mit den einhergehenden Gefahren gemindert wird.

Aufgabe der Erfindung ist es nunmehr, ein Verfahren zur Umhüllung eines solchen Implantats zu schaffen, das sich einfach umsetzen lässt und für die Anfertigung solcher Implantate im großen Stil taugt. Zudem ist es Aufgabe der Erfindung einen solcherart umhüllten Stent insofern zu verbessern, als er bei kostengünstiger Herstellung und einfacher Handhabung zur Vermeidung von Restenose beiträgt und thrombotisches Material mit großer Effizienz fixiert.

Diese Aufgaben werden durch die Verfahren nach Anspruch 1 und 22 und durch das Implantat nach Anspruch 11 gelöst. Vorteilhafte Ausführungsformen der Erfindung sind in den jeweiligen Unteransprüchen genannt.

Der wesentliche Grundgedanke der Erfindung liegt darin, die Ausnehmungen in der Oberfläche des Implantats mit einem Opfermaterial aufzufüllen und damit eine vorübergehend ebene Oberfläche zu schaffen. Auf dieser kann nachfolgend Abdeckmaterial abgeschieden werden, das eine filmartige Umhüllung ausbildet. Anschließend wird das Opfermaterial zumindest aus den Ausnehmungen, am besten von innen, wieder entfernt, so dass die Funktionalität des Implantats wieder hergestellt ist. Zurück bleibt der umhüllende Film, der auf der vom Gitter gebildeten Oberfläche ausreichenden Halt hat. Dabei ist für eine ausreichende Flexibilität des Filmes zu sorgen, da er die Ausdehnung des Implantats im Gefäß entsprechend mitmachen muss. Ein solche Flexibilität wird insbesondere durch eine netzartige Struktur erreicht, wobei die Dimension der Maschen um eine Größenordnung kleiner sein sollte, als die der Ausnehmungen. Im Übrigen wird nachfolgend der Begriff "Stent" synonym für derart röhrchenförmige Implantate gewählt. Die Erfindung ist auch auf vaskuläre Implantate, wie Filter, Stent Grafts oder sogenannte Occlusionsinstrumente bzw. Occluder anwendbar.

Wesentlich ist, dass nach der erfindungsgemäßen Lösung die filmartige Umhüllung mit den einzelnen Stegen der Gitterstruktur des Stents bzw. des Implantats nicht punktuell, sondern zumindest abschnittsweise ununterbrochen stoffschlüssig verbunden ist. Diese stoffschlüssige Verbindung wird bei einem röhrenförmigen Stent bzw. Implantat im einzelnen zwischen der die Außenseite des Stents bzw. Implantats bildenden Oberfläche der Gitterstruktur und der vorzugsweise metallischen filmartigen Umhüllung gebildet.

Die wesentlichen Schritte des erfindungsgemäßen Verfahrens sind zunächst das Aufschieben eines vorhandenen Stents auf eine zylindrische Halterung. So fixiert, wird in einem weiteren Schritt ein später wieder zu entfernendes Opfermaterial, insbesondere Kupfer, über dem Gitter des Stents abgeschieden. Dieses Abscheiden erfolgt so lange, bis das Opfermaterial die Ausnehmungen zumindest nahezu ausfüllt und die Gitterstruktur geschlossen ist. Wie zu beschreiben ist, kann es vorteilhaft sein, die Oberfläche insofern nachzubearbeiten, als das Opfermaterial von Außen zumindest teilweise abgenommen wird, bevor in einem weiteren Schritt die Abdeckschicht auf der so geschaffenen Oberfläche des Stents abgeschieden wird.

Je nach dem, wie stark die Verbindung zwischen dem Gitter des Stents und der Abdeckschicht sein soll, wird bei der Nachbearbeitung mehr oder weniger Opfermaterial von Außen abgenommen. So kann es vorteilhaft sein, durch die Nachbearbeitung die Gitterstruktur des Stents so weit freizulegen, dass die Abdeckschicht sich damit verbinden kann. Falls hingegen zwischen dem Gitter und der Abdeckschicht eine Schicht Opfermaterial verbleibt, wird die von der Abdeckschicht auf dem Stent gebildete Umhüllung wegen der nachher fehlenden Verbindung verschieblich.

Letztendlich wird das Opfermaterial, das sich zwischen Abdeckschicht und Stent und vor allem in den Ausnehmungen befindet, entfernt.

Ein wesentlicher Vorteil des erfindungsgemäßen Verfahrens und des daraus entstandenen Stents ist, dass das Einwachsen von Gewebe in das Gefäß, also die Bildung einer Restenose, zumindest drastisch reduziert, wenn nicht sogar völlig vermieden werden kann. Das Verfahren lässt sich auf alle bekannten Arten von Stents (bzw. vaskuläre Implantate) anwenden. Insofern lassen sich beispielsweise alle kommerziell erhältlichen Stents mit diesem Verfahren verbessern. Selbstverständlich ist das erfindungsgemäße Verfahren aber auch bei vaskulären Implantaten, wie beispielsweise Filter, Stent Grafts und Occlusionsinstrumenten, anwendbar, um diese medizinischen Vorrichtungen insbesondere im Hinblick auf ihre Körperverträglichkeit zu verbessern.

An dieser Stelle sei darauf hingewiesen, dass die hierin im Zusammenhang mit der Verbesserung eines Stents beschriebenen Ausführungsformen, Vorteile und Wirkungen des erfindungsgemäßen Verfahrens in analoger Weise auch bei vaskulären Implantaten, wie beispielsweise Filter, Stent Grafts oder Occlusionsinstrumenten, anwendbar bzw. erzielbar sind.

Ein weiterer mit dem erfindungsgemäßen Verfahren erzielbarer Vorteil ist darin zu sehen, dass - aufgrund der zuvor beschriebenen vollständigen Umhüllung des Stents (bzw. des vaskulären Implantats) mit der Abdeckschicht - auch die Möglichkeit besteht, durch eine geeignete Nachbearbeitung der Umhüllung, und insbesondere durch eine nachträgliche Strukturierung der Umhüllung, einen "gecoverten" Stent oder Stent Graft mit sehr feiner Maschenweite herzustellen, der ideal zur Abdeckung von Aneurysmen geeignet ist. Insbesondere besteht somit die Möglichkeit, den Stent oder das vaskuläre Implantat speziell für den Patienten anzupassen, indem der Umfang und/oder die Art der Nachbearbeitung des vollständig umhüllten Stents (bzw. Implantats) an den Einzelfall angepasst werden. Dabei sei angemerkt, dass das "Covering", also die den gecoverten Stent ausbildende Beschichtung, in der Regel nicht übermäßigen Kräften ausgesetzt ist; allerdings ist es von Vorteil, wenn das Covering möglichst dünn ist, um die Implantation und gegebenenfalls auch Explantation der medizinischen Vorrichtung beispielsweise mit einem Ein- bzw. Ausführbesteck zu ermöglichen, welches einen möglichst kleinen Durchmesser aufweist. Nach der Positionierung des gecoverten Stents oder Stent Grafts im Körper des Patienten kann das Covering mit einem konventionell hergestellten Stent aufgespannt werden, der über die entsprechenden makroskopischen Wanddicken und Stegbreiten verfügt, um die mechanische Stabilität des Implantates zu gewährleisten.

Im Hinblick auf eine mit dem erfindungsgemäßen Verfahren hergestellte medizinische Vorrichtung (Stent, vaskuläre Implantat) ist schließlich noch als Vorteil zu nennen, dass bei dieser medizinischen Vorrichtung - insbesondere infolge des umhüllenden Films, der auf der vom Gitter gebildeten Oberfläche ausreichenden Halt hat - eine durchgängige stoffschlüssige Verbindung zwischen der Gitterstruktur des konventionell hergestellten Implantats (Stents) und der Umhüllung hergestellt wird. Damit wird erreicht, dass die medizinische Vorrichtung gemäß der vorliegenden Erfindung deutlich weniger thrombogen ist als herkömmliche medizinische Vorrichtung, wie beispielsweise Stents oder Occlusionsinstrumente, die nach einem herkömmlichen Verfahren hergestellt sind.

Besonders vorteilhaft ist es, wenn die umhüllende Abdeckschicht als Film aus Metall, insbesondere aus einer Nickel-Titan-Legierung (NiTi), beispielsweise Nitinol, hergestellt wird. Dabei hat Metall den Vorteil, dass es durch Aufdampfen oder durch Aufsputtern in einstellbarer Stärke und mit hoher Oberflächengüte aufbringbar ist. Zudem kann eine chemisch und biologisch inerte Legierung, wie z.B. das genannte Nitinol, gewählt werden, die die Funktion des Organismus nicht beeinträchtigt. Als weiterer wesentlicher Vorteil der umhüllenden Metallfolie ist deren Stabilität zu nennen, durch die eine besonders ebene Oberfläche über der gitterartigen Struktur erreicht wird. Mit der stabilen und ebenen Oberfläche des Stents kann eine gute Fixierung eventuell vorhandenen thrombotischen Materials an der Innenwand des Gefäßes gewährleistet werden.

Alternativ oder zusätzlich zu der zuvor genannten vorteilhaften Weiterbildung des erfindungsgemäßen Verfahrens, bei welcher die umhüllende Abdeckschicht als Film aus Metall, insbesondere aus einer Nickel-Titan-Legierung (NiTi), beispielsweise Nitinol, hergestellt wird, ist es aber auch denkbar, dass durch Aufdampfen von biodegrdierbaren Metallfilmen, insbesondere aus Eisen, Eisenlegierungen, Magnesium oder Magnesiumlegierungen, auf dem Gitter der medizinischen Vorrichtung einen umhüllenden Film ("Covering") auszubilden, der sich nach einer bestimmten, vorzugsweise vorab festlegbaren Zeit zumindest teilweise oder vollständig zersetzt und somit auflöst. Damit kann die medizinische Vorrichtung besonders anwendungsbezogen verwendet werden.

In einer bevorzugten Realisierung der erfindungsgemäßen Lösung ist vorgesehen, dass das mit dem erfindungsgemäßen Verfahren zu bearbeitende (konventionell hergestellte) Implantat bzw. der mit dem erfindungsgemäßen Verfahren zu bearbeitende (konventionell hergestellte) Stent und/oder der mit dem erfindungsgemäßen Verfahren auf dem Gitter der medizinischen Vorrichtung (Implantat, Stent) aufzubringende bzw. aufgebrachte Metallfilm aus einem vorzugsweise biodegradierbaren Metall, insbesondere aus Eisen, Eisenlegierungen, Magnesium oder Magnesiumlegierungen besteht.

Selbstverständlich ist es aber auch denkbar, dass der Metallfilm zumindest teilweise aus einem röntgendichten Material, wie beispielsweise Gold, Platin, Niob oder Tantal, gebildet wird bzw. gebildet ist. Auch können diese Metalle teilweise beim Sputtern in den metallfilm eingebracht werden, so dass der Metallfilm beispielsweise aus ternären Legierungen NiTiTa oder NiTiNB besteht.

In einer vorteilhaften Ausführungsform wird die gitterartige Struktur des Stent mit einem Metall als Opfermaterial, insbesondere mit Kupfer, vermittels eins galvanischen Verfahrens aufgefüllt. Dabei wird der insbesondere aus Nitinol gefertigte Stent auf ein Rohr aus entsprechendem Metall, also insbesondere aus Kupfer, aufgespannt und in einem Bad galvanisch mit dem Metall überzogen. Der Prozess wird gestoppt, wenn die elektrochemisch abgeschiedene Schicht die Gitterstruktur weitgehend, insbesondere komplett, überdeckt. Die so entstandene Oberfläche wird vorteilhafterweise im Anschluss mit einem weichen Schleifpapier auf einer Drehbank zur Entfernung des überschüssigen Metalls nachbearbeitet. Im Anschluss daran wird das so "verstopfte" Gitter des Stents, in dessen Öffnungen das Kupfer abgeschieden ist, wie ein gewöhnliches Rohr mit Metall, insbesondere mit superelastischem NiTi, besputtert, bevor der Kupferkern durch eine selektive Säurebehandlung entfernt wird.

Als Opfermaterial kann auch Au, Cr, FeCo oder Ähnliches gewählt werden, das auf einem Substrat-Röhrchen aus einem Opfermaterial, vorzugsweise auf Cu, aber auch auf Au, Cr oder FeCo aufgebracht wird. Zu beachten ist, dass sich Substrat und galvanisch abgeschiedene Schicht selektiv gegen den Stent aus NiTi ätzen lassen, wobei das Opfersubstrat weicher als das Material des Stents sein sollte, damit durch das Abschleifen des Opfersubstrats kein Stent-Material und nur geringe Mengen des NiTi entfernt werden. Dabei besitzt NiTi eine größere Härte als Kupfer.

Die Verwendung von NiTi, insbesondere von Nitinol, zur Herstellung von Stents ist bekannt. Wie dargelegt, ist zur Umhüllung der Stents NiTi ebenso besonders zu bevorzugen. Dabei ist neben der Biokompatibilität das wesentliche Merkmal dieser Legierungen vor allem das Formgedächtnis und die superelastische Eigenschaft. So ist NiTi ein bevorzugter Vertreter der Formgedächtnis-Legierungen. Es ist zu mindestens 8% pseudoelastisch verformbar, korrosionsbeständig und hochfest. Innerhalb eines Temperaturintervalls und oberhalb einer charakteristischen Vorspannung von einigen hundert MPa hat die Spannungs-Dehnungs-Kurve ein Plateau. In diesem Dehnungsbereich tritt eine Phasenumwandlung innerhalb des Materials auf, wobei sich unter einer Belastung der kubisch flächenzentrierte Austenit in monoklinen Martensit umwandelt. Der spannungsinduzierte Martensit kann sich entsprechend der angelegten Spannung entzwillingen und ermöglicht so innerhalb des Plateaus eine Deformation des Materials bei konstanter Gegenkraft. Dabei können über die Phasentransformation im spannungsinduzierten Martensit Dehnungen bis etwa 8% abgefangen werden, ohne dass plastische Verformung eintritt. Bei der Entlastung des Martensits wandelt sich dieser wieder in den Ausgangszustand des Austenits um.

Wegen seiner Biokompatibilität wird NiTi in der Medizintechnik häufig eingesetzt. Dabei sind die superelastischen Eigenschaften des NiTi gerade bei medizinischen Werkzeugen, wie Kathetern insbesondere zur Positionierung von Stents, die während ihres Einsatzes im Körper starken Verformungen ausgesetzt werden, von Vorteil. Gerade Gewebespreizer mit superelastischen Eigenschaften haben den Vorteil, dass sie das Gewebe weniger stark schädigen als Spreizer aus anderen Materialien. Der Formgedächtniseffekt des NiTi lässt sich gerade bei den Stents nutzen. Diese werden bei Zimmertemperatur im martensitischen Zustand verformt. Ist nun bei Körpertemperatur die austenitische Hochtemperaturphase stabil, wandelt sich das Implantat zu seiner ursprünglichen Form. Somit können sich die zusammengefalteten Stents im Körper selbstständig entfalten. Das gilt natürlich auch für eine Ummantelung aus NiTi, welche dieselben elastischen Eigenschaften zeigt. Insofern bilden solche mit NiTi ummantelten NiTi-Stents ideale Implantate zur Gefäßerweiterung und Gefäßstützung.

Die Herstellung der dünnen Abdeckschicht mit superelastischem Verhalten erfolgt vorteilhafterweise durch eine physikalische Abscheidmethode, vorzugsweise durch Kathodenzerstäuben oder Sputtern. Dabei können derart gesputterte Filme ein superelastisches Verhalten bei Körpertemperatur mit einer Dehnung von mehr als 6,5% beispielweise bei einer Plateauspannung von 400 MPa oder weniger zeigen.

Wird die Flexibilität nicht durch das Material an sich ermöglicht oder reicht dessen intrinsische Flexibilität der Abdeckschicht nicht aus, ist es vorteilhaft, auch darin Ausnehmungen vorzusehen, die jedoch kleiner als die des Stents sein sollten. Dabei wird die Strukturierung der Abdeckschicht vorteilhafterweise mittels photolithographischer, lasergestützter oder anderer Prozesse vorgenommen. Damit ist es möglich eine Abdeckschicht von feiner netzartiger Struktur mit hoher Elastizität zu erzeugen. Eine besonders bevorzugte Anwendung des Verfahrens ist somit die Optimierung von NiTi-Zylinderstrukturen für endovaskuläre oder neurovaskuläre NiTi-Stents, wobei mit der Abdeckschicht eine Verringerung der Porengröße unter Beibehaltung der superelastischen Eigenschaften der Stützstruktur aus insbesondere lasergeschnittenem NiTi möglich ist.

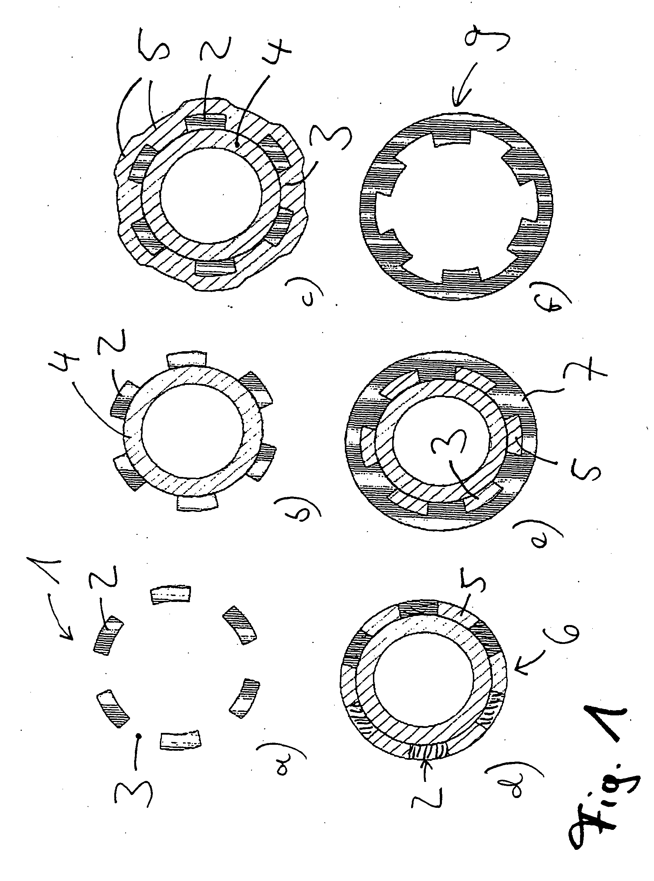

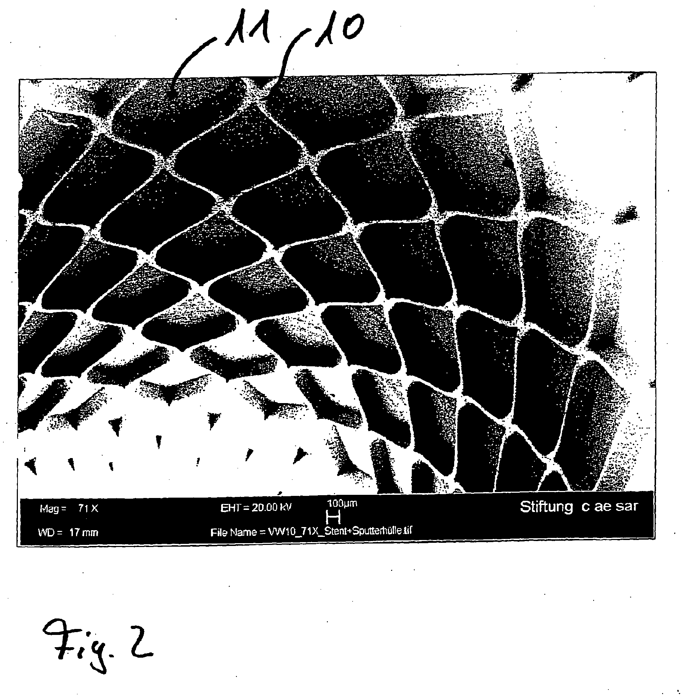

Nachfolgend wird die Erfindung anhand der Figuren 1 und 2 näher erklärt. Es zeigen:
- **Figur 1**: die Verfahrensschritte zur Ummantelung eines NiTi-Stents durch eine NiTi- Schicht unter Einsatz von Mikrogalvanik und Sputter-Abscheidung;
- **Figur 2**: eine REM-Aufnahme (Innenansicht) eines NiTi-Stent, der mit Hilfe des be- schriebenen Verfahrens mit einer NiTi-Einhüllenden besputtert ist; und
- **Figur 3**: die Verfahrensschritte zur Herstellen eines NiTi-Stents mit einer Innen- schicht und einer NiTi-Ummantelungsschicht unter Einsatz von Mikrogal- vanik und Sputter-Abscheidung.

Figur 1 (a) zeigt schematisch einen Schnitt durch einen NiTi-Stent 1, dessen Wandung 2 von den Ausnehmungen 3 unterbrochen ist. Dieser Stent zeigt bei Körpertemperatur superelastisches Verhalten. Durch Abkühlen des Stents in den martensitischen Zustand, beispielsweise durch Einsatz eines Kältesprays oder in flüssigem Stickstoff, kann der Stent aufgeweitet und auf eine zylindrische Halterung, die hier von einem Kupfer-Rohr 4 gebildet wird, aufgespannt werden. Um einen sicheren Halt zu gewährleisten, ist der Außendurchmesser des Kupfer-Rohres 4 größer als der Innendurchmesser des NiTi-Stents. Anschließendes Erwärmen des Stents auf Raumtemperatur führt zu einem formschlüssigen Kontakt zwischen dem aufgespanntem Stent und dem Substrat (Figur 1b).

Im Anschluss daran wird in einem mikrogalvanischem Prozess eine Kupferschicht 5 als Opfermaterial auf das Kupfer-Substrat 4 elektrochemisch abgeschieden. Das Abscheiden wird solange durchgeführt, bis der komplette Stent mit Kupfer bedeckt ist und das Opfermaterial die Ausnehmungen 3 auffüllt (Figur 1c). Anschließend wird das den NiTi-Stent 1 überwölbende Kupfer 5 abgetragen. Das kann manuell mit feinem Schmirgelpapier auf einer Drehbank geschehen. Je nach dem wird so viel Kupfer abgetragen, bis die Wandung 2 des NiTi-Stent in der aufgewachsenen Cu-Schicht 5 freigelegt ist und ein Zylinder 6 mit gleichmäßiger Wandstärke entstanden ist (Figur 1d).

Nun wird in diesem Fall mit der Sputtertechnik eine dünne NiTi- Abdeckschicht 7 auf dem so entstandenen Zylinder 6, der von dem mit Cu ausgefüllten Stent 1 gebildet wird, abgeschieden (Figur 1e). In diesem Fall verbindet sich die NiTi- Abdeckschicht 7 mit der Wandung 2 des Stent. Der Gesamtdurchmesser nimmt dabei zwischen 10 und 100 µm zu, was einer Wandstärke des NiTi zwischen 5 und 50 µm entspricht. Zuletzt wird die zylindrische Halterung 4 und das in den Ausnehmungen 3 befindliche Opfermaterial 5 mittels eines selektiven Ätzmediums, z.B. 40% HNO₃, entfernt so dass nur noch der ummantelte Stent 9 verbleibt. (Figur 1f). Das selektive Ätzen geschieht vorteilhafterweise durch den Einsatz einer Säurepumpe, die das Ätzmedium durch den Kupfer-Zylinder 4 pumpt und den Kern auflöst. Typische Ätzzeiten liegen im Bereich von 10-30 Minuten, typische Wandstärken des Kupfer-Röhrchens bei 0,5 mm bei einem Außendurchmesser von 5 mm.

Auf diese Weise lassen sich insbesondere NiTi-Stents mit einem Durchmesser von 4.5 mm und einer Wandstärke von 0.2 mm mit einer NiTi-Ummantelung von 15 µm beschichten. Mit gängigen photolithographischen und nasschemischen Ätzverfahren ist es nun möglich, die aufgewachsene NiTi-Schicht zu strukturieren, beispielsweise unter Einsatz eines Photolacks und einem selektiven Ätzmittel. Zum Entfernen des Opferkerns kann als selektives Ätzmediums HNO₃, Fe₃Cl oder Ammoniumperoxidsulfat-Lösung verwendet werden.

Das Verfahren lässt sich besonders vorteilhaft einsetzen bei Stents, die einen Durchmesser zwischen 100 µm und 100 mm, insbesondere zwischen 1 mm und 36 mm, und eine Wandstärke zwischen 50 µm bis 5 mm, insbesondere zwischen 50 µm bis 600 µm haben. Die Dicke der NiTi Ummantelung ist zwischen 1 und 100 µm, wobei der Bereich zwischen 5 bis 50 µm zu bevorzugen ist.

Figur 2 zeigt eine elektronenmikroskopische Aufnahme der Wandung eines erfindungsgemäß beschichteten Stents. Zu erkennen sind die Streben 10 der netzartigen Struktur, deren Maschen von Außen mit der NiTi-Ummantelung 11 bedeckt sind. Unten ist ein Maßstab eingezeichnet, wobei der Balken eine Länge von 100 µm aufweist. Die Wandstärke, also die Breite der Streben 10 ist etwa in diesem Bereich. Ebenso zu erkennen ist, dass die die von den Streben 10 gebildete Oberfläche bedeckende NiTi-Ummantelung 11 zumindest nahezu geschlossen ist.

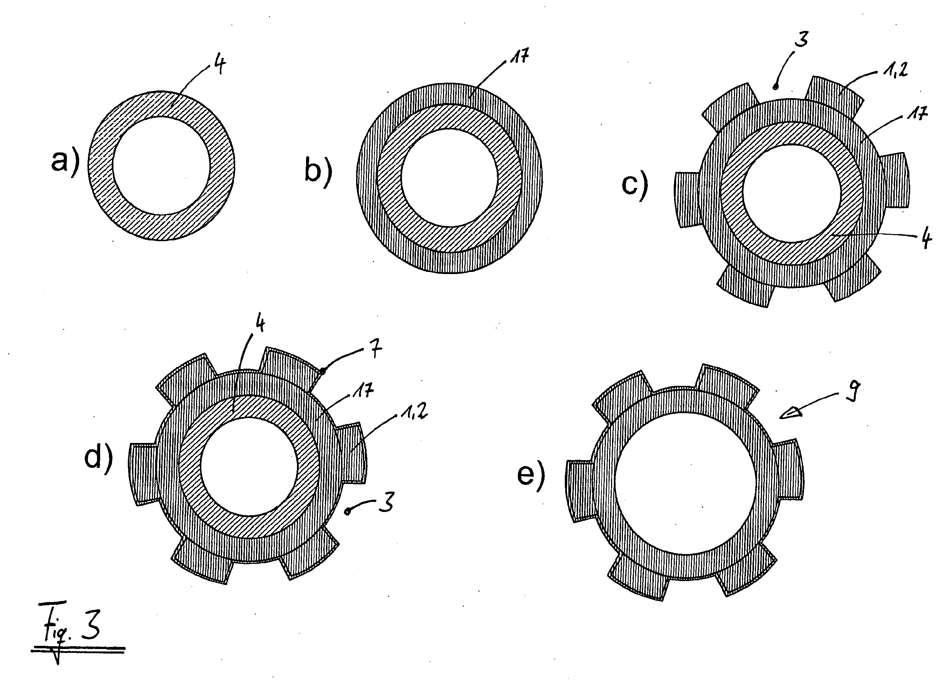

Figur 3 zeigt die Verfahrensschritte zur Herstellen eines NiTi-Stents 9, welcher im fertiggestellten Zustand zusätzlich zu der zuvor unter Bezugnahme auf Figur 1 beschriebenen NiTi-Ummantelungsschicht bzw. Abdeckschicht 7, welche nachfolgend auch als "erste Metallschicht 7" bezeichnet wird, eine weitere Schicht 17 aufweist. Diese weitere Schicht 17 wird nachfolgend auch als "zweite Metallschicht 17" bezeichnet und besteht vorzugsweise aus einem Metall, insbesondere aus einer Nickel-Titan-Legierung. Bei dem in Figur 3 gezeigten Verfahren kann, wie auch bei dem in Figur 1 gezeigten Verfahren, die Mikrogalvanik und Sputter-Abscheidung zum Einsatz kommen.

Im einzelnen zeigt Figur 3a schematisch einen Schnitt durch ein in diesem exemplarischen Fall zylindrisches Substrat 4, welches als Halterung dient. In einer bevorzugten Realisierung des in Figur 3 gezeigten Verfahrens kann diese Halterung mit einem Kupfer-Rohr 4 gebildet sein, wobei das Kupfermaterial als Opfermaterial für die spätere Mikrogalvanik dient. Es ist ersichtlich, dass die Erfindung nicht auf ein zylinderförmiges Substrat 4 beschränkt ist; vielmehr sollte die Formgebung des Substrats an die Formgebung des herzustellenden Stents 9 entsprechend angepasst sein.

Auf die Außenseite des in Figur 3(a) gezeigten zylindrischen Substrats 4 wird im nächsten Verfahrensschritt eine Metallschicht 17, vorzugsweise eine NiTi-Schicht ausgebildet, wie es in Figur 3(b) gezeigt ist. Hierfür können geeignete Verfahren verwendet werden, wie beispielsweise ein Aufdampfverfahren oder eine Sputter-Abscheidung. Selbstverständlich wäre es aber auch denkbar, dass die zweite Metallschicht 17 als Hülse über das zylindrische Substrat 4 gestülpt wird. Vorzugsweise sollte das verwendete Verfahren zur Ausbildung der zweiten Metallschicht 17 an der Außenseite des Substrats 4 ausgelegt sein, dass die Schichtdicke der zweiten Metallschicht 17 vorgebbar ist. Abhängig von der geplanten Anwendung des fertig gestellten Stents 9 weist die zweite Metallschicht 17 eine Stärke zwischen 1 µm und 10 mm, und vorzugsweise zwischen 5 bis 50 µm, auf.

Anschließend wird ein handelsüblicher Stent 1 über die auf der zylindrischen Halterung 4 angebrachten zweiten Metallschicht 17 gezogen. Figur 3(c) zeigt schematisch einen Schnitt durch einen NiTi-Stent 1, dessen Wandung 2 von den Ausnehmungen 3 unterbrochen ist. Dieser Stent 1 zeigt bei Körpertemperatur superelastisches Verhalten. Durch Abkühlen des Stents in den martensitischen Zustand, beispielsweise durch Einsatz eines Kältesprays oder in flüssigem Stickstoff, kann der Stent 1 aufgeweitet und auf die auf der zylindrischen Halterung 4 ausgebildeten zweiten Metallschicht 17 aufgespannt werden. Um einen sicheren Halt zu gewährleisten, ist vorzugsweise der Außendurchmesser der zweiten Metallschicht 17 größer als der Innendurchmesser des NiTi-Stents 1.

Anschließendes Erwärmen des Stents 1 auf Raumtemperatur führt zu einem formschlüssigen Kontakt zwischen dem aufgespanntem Stent 1 und der zweiten Metallschicht 17 (Figur 3c).

Im Anschluss daran wird in diesem Fall mit der Sputtertechnik eine dünne NiTi- Abdeckschicht 7 auf die Außenseite des aufgespannten Stents 1 abgeschieden (Figur 3d). In diesem Fall verbindet sich die NiTi- Abdeckschicht 7 einerseits mit der Wandung 2 des Stent und andererseits mit den in den Ausnehmungen 3 freiliegenden Abschnitten der zweiten Metallschicht 17. Der Gesamtdurchmesser nimmt dabei zwischen 10 µm und 100 µm zu, was einer Wandstärke der ersten Metallschicht 7 zwischen 5 und 50 µm entspricht.

Zuletzt wird die zylindrische Halterung 4 mittels eines selektiven Ätzmediums, z.B. 40% HNO₃, entfernt, so dass nur noch der in Figur 3(e) gezeigte Stent 9 verbleibt, der die Ni-Ti-Ummantelungsschicht bzw. Abdeckschicht 7 und die innere NiTi-Schicht 17 aufweist. Das selektive Ätzen geschieht erneut in vorteilhafter Weise durch den Einsatz einer Säurepumpe, die das Ätzmedium durch den Kupfer-Zylinder 4 pumpt und den Kern auflöst. Typische Ätzzeiten liegen im Bereich von 10 bis 30 Minuten, typische Wandstärken des Kupfer-Röhrchens bei 0,5 mm bei einem Außendurchmesser von 5 mm.

Auf diese Weise lassen sich insbesondere NiTi-Stents mit einem Durchmesser von 4.5 mm und einer Wandstärke von 0.2 mm mit einer NiTi-Außenummantelung von 15 µm und einer NiTi-Innenummantelung von 1 µm bis 10 mm beschichten.

Mit gängigen photolithographischen und nasschemischen Ätzverfahren ist es nun möglich, die aufgewachsene NiTi-Aussenschicht 7 oder gegebenenfalls auch die NiTi-Innenschicht 17 zu strukturieren, beispielsweise unter Einsatz eines Photolacks und einem selektiven Ätzmittel. Zum Entfernen des Opferkerns kann als selektives Ätzmediums HNO₃, Fe₃Cl oder Ammoniumperoxidsulfat-Lösung verwendet werden.

Ein Stent 9, welcher, wie in Figur 3(e) gezeigt, einen innenliegenden Metallfilm 17 aufweist, zeichnet sich dadurch aus, dass er unter anderem eine äußerst glatte Innenoberfläche aufweist, so dass durch den Stent im implantierten Zustand die Körperflüssigkeit besonders widerstandsarm und insbesondere ohne Wirbelbildung fließen kann. Von daher ist die Emboliegefahr weiter reduziert. Darüber hinaus weist ein Stent mit einem innenliegenden Metallfilm insgesamt Vorteile im Hinblick auf eine sichere Positionierung des Implantates im Körper des Patienten auf, da das Problem von auftretenden Spannungen etc. im Implantat reduziert werden kann. Denkbar wäre es ferner, dass in der innenliegenden Schicht, welche vorzugsweise ein Metallfilm ist, aber auch aus einer Kunststoff- oder Polymerkomposition gebildet sein kann, Medikamente etc. aufgenommen sind, welche im implantierten Zustand des Stents über einen längeren Zeitraum kontinuierlich abgegeben werden.

Grundsätzlich gilt, dass - aufgrund der zur Herstellung der Schichten verwendeten Dünnschichttechnik - sowohl der ummantelnde Metallfilm, als auch der innenliegenden Metallfilm sehr rein sind und daher auch besonders biokompatibel erscheinen.

Die Erfindung ist nicht auf die in den Figuren gezeigten speziellen Ausführungsformen, und insbesondere die dargestellten röhrchenförmigen Implantate beschränkt. Vielmehr kann das erfindungsgemäße Verfahren beispielsweise auch bei flachen Gewebestrukturen entsprechend angewandt werden. Derartige Gewebestücke lassen sich nach deren Behandlung anschließend beispielsweise durch eine entsprechende Umformung in die endgültige Form des Implantates bringen.

Auch ist die Erfindung nicht allein auf die Herstellung von Stents beschränkt. Vielmehr kann das erfindungsgemäße Verfahren auch auf vaskuläre Implantate, wie Filter, Stent Grafts oder Occlusionsinstrumente, angewandt werden. Ferner kann anstelle einer NiTi-Innenschicht eine aus einer Kunststoff- oder Polymerkomposition bestehende Schicht verwendet werden.

## Patentansprüche

1. Verfahren zur Aufbringung einer umhüllenden Abdeckschicht (7) auf einem röhrchenförmigen endoluminalen Implantat (1), wobei die Oberfläche des Implantats (1) von einer Vielzahl von Ausnehmungen (3) durchbrochen ist, wobei die Abdeckschicht (7) durch Abscheidung von Material auf der Oberfläche des Implantats (1) erzeugt wird,
**dadurch gekennzeichnet, dass**
- in einem ersten Schritt das Implantat (1) auf eine zylindrische Halterung (4) aufgeschoben wird,
- in einem zweiten Schritt ein Opfermaterial (5), auf der Oberfläche des Implantats (1) abgeschieden wird, bis das abgeschiedene Opfermaterial (5) die Ausnehmungen (3) zumindest nahezu ausfüllt,
- in einem dritten Schritt die Abdeckschicht (7) auf der Oberfläche des mit Opfermaterial (5) versehenen Implantats (1) abgeschieden, und dass
- in einem vierten Schritt die zylindrische Halterung (4) und das in den Ausnehmungen (3) befindliche Opfermaterial (5) entfernt wird.

2. Verfahren nach Anspruch 1,
**dadurch gekennzeichnet, dass**
in einem Zwischenschritt zwischen dem zweiten und dem dritten Schritt die Oberfläche des mit Opfermaterial (5) versehenen Implantats (1) nachgearbeitet wird, wobei das Opfermaterial (5) von Außen zumindest teilweise abgenommen wird.

3. Verfahren nach Anspruch 2,
**dadurch gekennzeichnet, dass**
das Opfermaterial (5) soweit abgenommen wird, bis die Oberfläche des Implantats (1) vom Opfermaterial (5) befreit ist.

4. Verfahren nach einem der vorherigen Ansprüche,
**dadurch gekennzeichnet, dass**
auch die Abdeckschicht (7) als unterbrochene Struktur aufgebracht wird, wobei die Dimension der Ausnehmungen um eine Größenordnung kleiner ist, als die in der Oberfläche des Implantats (1) befindlichen Ausnehmungen (3).

5. Verfahren nach einem der vorherigen Ansprüche,
**dadurch gekennzeichnet, dass**
als Abdeckschicht (7) ein Film aus Metall aufgebracht wird.

6. Verfahren nach Anspruch 5,
**dadurch gekennzeichnet, dass**
eine Nickel-Titan-Legierung aufgesputtert wird.

7. Verfahren nach einem der vorherigen Ansprüche,
**dadurch gekennzeichnet, dass**
das Opfermaterial (5) in einem galvanischen Verfahren aufgebracht wird.

8. Verfahren nach einem der vorherigen Ansprüche,
**dadurch gekennzeichnet, dass**
die zylindrische Halterung (4) und das Opfermaterial (5) mittels einer selektiven Säurebehandlung entfernt wird.

9. Verfahren nach einem der vorherigen Ansprüche,
**dadurch gekennzeichnet,dass**
das Implantat (1) im ersten Schritt auf eine Halterung (4) aus Metall aufgeschoben wird.

10. Verfahren nach einem der vorherigen Ansprüche,
**dadurch gekennzeichnet, dass**
das Implantat auf die Halterung (4) aufgespannt wird, wobei es unter Dehnung auf die Halterung (4) aufgeschoben und nachfolgend entspannt wird.

11. Endoluminales Implantat (9) mit einer Gitterstruktur (1, 2) und einer ersten Metallschicht (7), wobei die Gitterstruktur (1, 2) stoffschlüssig durchgehend mit der ersten Metallschicht (7) verbunden ist.

12. Implantat (9) nach Anspruch 11,
**dadurch gekennzeichnet, dass**
die erste Metallschicht (7) als eine Abdeckschicht (7) ausgebildet ist, welche eine Außenseite der Gitterstruktur (1, 2) des Implantats (9) umgibt.

13. Implantat (9) nach Anspruch 12,
**dadurch gekennzeichnet, dass**
die Abdeckschicht (7) aus einer Nickel-Titan-Legierung gebildet ist.

14. Implantat (9) nach einem der Ansprüche 11 bis 13,
**dadurch gekennzeichnet, dass**
die Gitterstruktur (1, 2) des Implantats (9) ein herkömmlicher Stent (1) mit einem Durchmesser zwischen 100 µm und 100 mm, und vorzugsweise zwischen 1 und 36 mm, ist.

15. Implantat (9) nach Anspruch 14,
**dadurch gekennzeichnet, dass**
der Stent (1) eine Wandstärke zwischen 50 µm bis 5 mm, und vorzugsweise zwischen 50 µm bis 600 µm, aufweist.

16. Implantat (9) nach einem der Ansprüche 11 bis 15,
**dadurch gekennzeichnet, dass**
die erste Metallschicht (7) eine Stärke zwischen 1 und 100 µm, vorzugsweise zwischen 5 bis 50 µm, aufweist.

17. Implantat (9) nach einem der Ansprüche 11 bis 16,
**dadurch gekennzeichnet, dass**
das Implantat (9) ferner eine zweite Metallschicht (17) aufweist, welche als eine die Innenseite der Gitterstruktur (1, 2) des Implantats (9) umgebende Schicht ausgebildet ist.

18. Implantat (9) nach Anspruch 17,
**dadurch gekennzeichnet, dass**
die zweite Metallschicht (17) an der Innenseite der Gitterstruktur (1, 2) durchgehend anliegt.

19. Implantat (9) nach Anspruch 17 oder 18,
**dadurch gekennzeichnet, dass**
die zweite Metallschicht (17) mit der ersten Metallschicht (7) abschnittsweise stoffschlüssig verbunden ist.

20. Implantat (9) nach einem der Ansprüche 17 bis 19,
**dadurch gekennzeichnet, dass**
die zweite Metallschicht (17) aus einer Nickel-Titan-Legierung gebildet ist.

21. Implantat (9) nach einem der Ansprüche 17 bis 20,
**dadurch gekennzeichnet, dass**
die zweite Metallschicht (17) eine Stärke zwischen 1 µm und 10 mm, vorzugsweise zwischen 5 µm bis 50 µm, aufweist.

22. Verfahren zum Herstellen eines endoluminalen Implantats (9), bei dem zumindest eine Schicht (7, 17) mit einer Gitterstruktur (1, 2) durch ein PVD-Verfahren oder CVD-Verfahren verbunden wird, wobei eine filmartige Umhüllung gebildet wird, die mit einzelnen Stegen der Gitterstruktur (1, 2) zumindest abschnittsweise ununterbrochen staffschlüssig verbunden wird.

23. Verfahren nach Anspruch 22,
**dadurch gekennzeichnet, dass**
eine erste Schicht (7) der zumindest einen Schicht (7, 17) auf einer Außenseite der Gitterstruktur (1, 2) abgeschieden und dabei mit dieser stoffschlüssig verbunden wird.

24. Verfahren nach Anspruch 23,
**dadurch gekennzeichnet, dass**
eine zweite Schicht (17) der zumindest einen Schicht (7, 17) auf einer Innenseite der Gitterstruktur (1, 2) angeordnet wird, und dass die erste Schicht (7) derart auf der Außenseite der Gitterstruktur (1, 2) abgeschieden wird, dass zumindest abschnittsweise die zweite Metallschicht (17) mit der ersten Metallschicht (7) stoffschlüssig verbunden wird.

## Claims

1. Method for applying a coating layer (7) to a tubular intraluminal implant (1),
wherein the surface of the implant (1) is perforated by a plurality of apertures (3), wherein the coating layer (7) is produced by deposition of material onto the surface of the implant (1), **characterised in that**
- in a first step the implant (1) is pushed onto a cylindrical holder (4),
- in a second step, a sacrificial material (5) is deposited on the surface of the implant (1) until the deposited sacrificial material (5) at least almost fills the apertures (3),
- in a third step, the coating layer (7) is deposited onto the surface of the implant (1) provided with sacrificial material (5), and
- in a fourth step, the cylindrical holder (4) and the sacrificial material (5) situated in the apertures (3) is removed.

2. Method according to claim 1, **characterised in that**, in an intermediate step between the second step and the third step, the surface of the implant (1) provided with sacrificial material (5) is treated, wherein the sacrificial material (5) is at least partially removed from outside.

3. Method according to claim 2, **characterised in that** the sacrificial material is removed to such an extent that the surface of the implant (1) are freed from the sacrificial material (5).

4. Method according to one of the preceding claims, **characterised in that** the coating layer (7) is applied as an uninterrupted structure, wherein the dimension of the apertures is smaller by an order of magnitude than the apertures (3) situated in the surface of the implant (1).

5. Method according to one of the preceding claims, **characterised in that** a film of metal is applied as the coating layer (7).

6. Method according to claim 5, **characterised in that** a nickel-titanium alloy is sputter deposited.

7. Method according to one of the preceding claims, **characterised in that** the sacrificial material (5) is applied in a galvanic method.

8. Method according to one of the preceding claims, **characterised in that** the cylindrical holder (4) and the sacrificial material (5) are removed by means of a selective acid treatment.

9. Method according to one of the preceding claims, **characterised in that**, in the first step, the implant (1) is pushed onto a holder (4) made of metal.

10. Method according to one of the preceding claims, **characterised in that** the implant is mounted on the holder (4), wherein it is pushed onto the holder (4) while being stretched and is then relaxed.

11. Endoluminal implant (9) which has a mesh structure (1, 2) and a first metal layer (7), wherein the mesh structure (1, 2) is bonded throughout, with material contact, to the first metal layer (7).

12. Implant (9) according to claim 11, **characterised in that** the first metal layer (7) is configured as a coating layer (7) which surrounds an outside of the mesh structure (1, 2) of the implant (9).

13. Implant (9) according to claim 12, **characterised in that** the coating layer (7) comprises a nickel-titanium alloy.

14. Implant (9) according to one of the claims 11 to 13, **characterised in that** the mesh structure (1, 2) of the implant (9) is a conventional stent (1) with a diameter in the range of 100 µm to 100 mm, and preferably 1 mm to 36 mm.

15. Implant (9) according to claim 14, **characterised in that** the stent (1) has a wall thickness in the range of 50 µm to 5 mm and preferably 50 µm to 600 µm.

16. Implant (9) according to one of the claims 11 to 15, **characterised in that** the first metal layer (7) has a thickness in the range of 1 µm to 100 µm and preferably 5 µm to 50 µm.

17. Implant (9) according to one of the claims 11 to 16, **characterised in that** the implant (9) also comprises a second metal layer (17) which is configured as a layer surrounding the inside of the grid structure (1, 2) of the implant (9).

18. Implant (9) according to claim 17, **characterised in that** the second metal layer (17) lies against the inside of the mesh structure (1, 2) throughout.

19. Implant (9) according to claim 17 or 18, **characterised in that** the second metal layer (17) is bonded at least in sections to the first metal layer (7) with material contact.

20. Implant (9) according to one of the claims 17 to 19, **characterised in that** the second metal layer (17) comprises a nickel-titanium alloy.

21. Implant (9) according to one of the claims 17 to 20, **characterised in that** the second metal layer (17) has a thickness in the range of 1 µm to 10 mm, and preferably 5 µm to 50 µm.

22. Method for producing an endoluminal implant (9) wherein at least one layer (7, 17) is bonded to a mesh structure (1, 2) by means of a PVD process or a CVD process, whereby a film-like covering is made which is firmly bonded to the individual webs of the mesh structure (1, 2) at least in sections with uninterrupted material contact.

23. Method according to claim 22, **characterised in that** a first layer (7) of the at least one layer (7, 17) is deposited on an outside of the mesh structure (1, 2) and is therein bonded to it with material contact.

24. Method according to claim 23, **characterised in that** a second layer (17) of the at least one layer (7, 17) is arranged on an inside of the mesh structure (1, 2) and that the first layer (7) is deposited onto the outside of the mesh structure (1, 2) such that at least in sections, the second metal layer (17) is bonded to the first metal layer (7) with material contact.

## Revendications

1. Procédé d'application d'une couche de recouvrement (7) enveloppante sur un implant endoluminal (1) tubulaire, dans lequel la surface de l'implant (1) est ajourée par une pluralité d'évidements (3), dans lequel la couche de recouvrement (7) est générée par un dépôt de matériau sur la surface de l'implant (1),
**caractérisé en ce que**,
- dans une première étape, on fait glisser l'implant (1) sur un support cylindrique (4),
- dans une deuxième étape, un matériau sacrificiel (5) vient se déposer sur la surface de l'implant (1) jusqu'à ce que le matériau sacrificiel déposé (5) remplisse les évidements au moins quasiment en totalité (3),
- dans une troisième étape, la couche de recouvrement (7) vient se déposer sur la surface de l'implant (1) pourvu de matériau sacrificiel (5), et **en ce que**,
- dans une quatrième étape, le support cylindrique (4) et le matériau sacrificiel (5) se trouvant dans les évidements (3) sont enlevés.

2. Procédé selon la revendication 1,
**caractérisé en ce que**,
dans une étape intermédiaire située entre la deuxième et la troisième étape, la surface de l'implant (1) pourvu de matériau sacrificiel (5) fait l'objet d'un post-traitement, par lequel on enlève le matériau sacrificiel (5), au moins partiellement, à partir de l'extérieur.

3. Procédé selon la revendication 2,
**caractérisé en ce que**
le matériau sacrificiel (5) est retiré jusqu'à ce que la surface de l'implant (1) soit libérée du matériau sacrificiel (5).

4. Procédé selon l'une quelconque des revendications précédentes,
**caractérisé en ce que**
l'on applique également la couche de recouvrement (7) sous la forme d'une structure ajourée, dans lequel la dimension des évidements est inférieure d'un ordre de grandeur à celle des évidements (3) se trouvant dans la surface de l'implant (1).

5. Procédé selon l'une quelconque des revendications précédentes,
**caractérisé en ce que**,
l'on applique un film de métal en tant que couche de recouvrement (7).

6. Procédé selon la revendication 5,
**caractérisé en ce que**
l'on procède à une pulvérisation cathodique d'un alliage de nickel-titane.

7. Procédé selon l'une quelconque des revendications précédentes,
**caractérisé en ce que**
le matériau sacrificiel (5) est appliqué par un procédé galvanique.

8. Procédé selon l'une quelconque des revendications précédentes,
**caractérisé en ce que**
le support cylindrique (4) et le matériau sacrificiel (5) sont enlevés au moyen d'un traitement d'acide sélectif.

9. Procédé selon l'une quelconque des revendications précédentes,
**caractérisé en ce que**
l'on fait glisser l'implant (1), dans la première étape, sur un support (4) en métal.

10. Procédé selon l'une quelconque des revendications précédentes,
**caractérisé en ce que**
l'implant est étendu sur le support (4), dans lequel on le fait glisser sur le support (4) en l'étirant et dans lequel on le détend ensuite.

11. Implant endoluminal (9) comprenant une structure grillagée (1, 2) et une première couche métallique (7), dans lequel la structure grillagée (1, 2) est reliée à la première couche métallique en continu par liaison de matière. (7).

12. Implant (9) selon la revendication 11,
**caractérisé en ce que**
la première couche métallique (7) se présente sous la forme d'une couche de recouvrement (7), qui entoure une surface externe de la structure grillagée (1, 2) de l'implant (9).

13. Implant (9) selon la revendication 12,
**caractérisé en ce que**
la couche de recouvrement (7) est constituée d'un alliage de nickel-titane.

14. Implant (9) selon l'une quelconque des revendications 11 à 13,
**caractérisé en ce que**
la structure grillagée (1, 2) de l'implant (9) est un stent traditionnel (1) d'un diamètre compris entre 100 µm et 100 mm et, de préférence, entre 1 et 36 mm.

15. Implant (9) selon la revendication 14,
**caractérisé en ce que**
le stent (1) présente une épaisseur de paroi compris entre 50 µm et 5 mm, de préférence, entre 50 µm et 600 µm.

16. Implant (9) selon l'une quelconque des revendications 11 à 15,
**caractérisé en ce que**
la première couche métallique (7) présente une épaisseur comprise entre 1 et 100 µm, de préférence entre 5 et 50 µm.

17. Implant (9) selon l'une quelconque des revendications 11 à 16,
**caractérisé en ce que**
l'implant (9) présente, en outre, une seconde couche métallique (17) qui se présente sous la forme d'une couche entourant la surface intérieure de la structure grillagée (1, 2) de l'implant (9).

18. Implant (9) selon la revendication 17,
**caractérisé en ce que**
la seconde couche métallique (17) juxtapose et touche la surface intérieure de la structure grillagée en continu. (1, 2).

19. Implant (9) selon la revendication 17 ou 18,
**caractérisé en ce que**
la seconde couche métallique (17) est reliée, par sections, à la première couche métallique (7) par liaison de matière.

20. Implant (9) selon l'une quelconque des revendications 17 à 19,
**caractérisé en ce que**
la seconde couche métallique (17) est constituée d'un alliage de nickel-titane.

21. Implant (9) selon l'une quelconque des revendications 17 à 20,
**caractérisé en ce que**
la seconde couche métallique (17) présente une épaisseur comprise entre 1 µm et 10 mm, de préférence entre 5 µm et 50 µm.

22. Procédé de fabrication d'un implant endoluminal (9), dans lequel au moins une couche (7, 17) est reliée à une structure grillagée (1, 2) par un procédé PVD ou CVD, dans lequel une enveloppe ressemblant à un film est constituée, laquelle est reliée en continu, au moins par sections, à des barreaux individuels de la structure grillagée par liaison de matière (1, 2).

23. Procédé selon la revendication 22,
**caractérisé en ce que**
une première couche (7) de la au moins une couche (7, 17) vient se déposer sur une surface externe de la structure grillagée (1, 2) et se relie ainsi à celle-ci par liaison de matière

24. Procédé selon la revendication 23,
**caractérisé en ce que**
une seconde couche (17) de la au moins une couche (7, 17) est disposée sur une surface intérieure de la structure grillagée (1, 2) et **en ce que**
la première couche (7) vient se déposer sur la face externe de la structure grillagée (1, 2) de sorte que la seconde couche métallique (17) soit reliée, au moins par sections, à la première couche métallique (7) par liaison de matière.
